# EUROPEAN PATENT APPLICATION

(11) **EP 4 268 888 A1**
(43) Date of publication of application: **01.11.2023**
(21) Application number: 21908649.3
(22) Date of filing: 30.08.2021
(51) Int. Cl.: A61N 7/00

(54) **WATER PATH ARRANGEMENT STRUCTURE OF ULTRASOUND TREATMENT DEVICE, AND ULTRASOUND TREATMENT DEVICE**

(30) Priority: 24.12.2020 CN 202011551309
(71) Applicant: Chongqing Haifu Medical Technology Co., Ltd., Chongqing 400714 (CN)
(72) Inventor: ZOU, Ying, Chongqing 400714 (CN); SUN, Minyi, Chongqing 400714 (CN); CHENG, Zhengming, Chongqing 400714 (CN); LIU, Xiaolin, Chongqing 400714 (CN)
(74) Representative: Cohausz & Florack
(86) International application number: PCT/CN2021/115350
(87) International publication number: WO 2022/134646

(57) **Abstract**

The present disclosure relates to the technical field of ultrasound treatment, and in particular relates to a water path arrangement structure for an ultrasound treatment device, and an ultrasound treatment device. The water path arrangement structure for an ultrasound treatment device includes an ultrasound treatment head, a water supply pipeline, and a water receiving tray. The ultrasound treatment head is provided with a cavity configured to accommodate a breast for treatment and having a cavity inner wall with an arc surface, a first connecting channel in communication with the water supply pipeline is disposed at a bottom of the ultrasound treatment head, and during ultrasound treatment, a water medium enters the cavity from the bottom of the ultrasound treatment head and overflows from a top of the cavity of the ultrasound treatment head. The water receiving tray is disposed around a periphery of the ultrasound treatment head and configured to receive the water medium overflowing from the cavity of the ultrasound treatment head. Beneficial effects of the present disclosure lie in that: the ultrasound treatment head is directly used as a carrier for containing the water, so that water consumption and pipeline arrangement are reduced, tangling and interference are avoided, a simple structure and a compact layout are obtained, and the treatment operation becomes simpler, more convenient, and more flexible.

## Description

### TECHNICAL FIELD

The present disclosure relates to the technical field of ultrasound treatment, and in particular relates to a water path arrangement structure for an ultrasound treatment device, and an ultrasound treatment device.

### BACKGROUND

For current High Intensity Focused Ultrasound (HIFU) devices, a treatment head is generally immersed in a separate water tank, so that water is used as a transmission medium of ultrasound and cools the skin tissues through which the ultrasound passes. Since the whole treatment head is completely immersed in the water tank and a movement stroke needs to be reserved for the treatment head, the water tank generally has a large size and correspondingly needs a great deal of water.

Generally, when a HIFU treatment head is used for treating a breast disease, a breast at a treatment side is pulled towards one side with a surgical adhesive plaster to expose the skin of the affected breast part. In this case, a monitoring probe is disposed at a bottom of the treatment head so that tumor monitoring of the breast in each position and quadrant can be implemented without rotating the treatment head. However, when a spherical sound collection treatment head is used for treating the breast disease, if the skin of the affected breast part is exposed by pulling the breast with the surgical adhesive plaster, the ultrasound used for treatment will be blocked in a large area to generate heat, which will cause safety accidents. Therefore, the patient usually takes a prone position to keep the breasts naturally drooping. In this case, if the monitoring probe is disposed at the bottom of the treatment head, it is hard to obtain a relatively ideal monitoring angle all the time. Therefore, the monitoring probe is preferably arranged on a sidewall of the treatment head so that the treatment head is rotated to drive rotation of the monitoring probe to implement tumor monitoring of the breast in each position and quadrant. However, rotation of the treatment head may involve tangling and interference of some pipelines, which may affect the treatment effect of the treatment head.

### SUMMARY

In view of the above disadvantages of the prior art, an object of the present disclosure is to provide a water path arrangement structure for an ultrasound treatment device and an ultrasound treatment device which can solve the problems of large water consumption and inconvenient treatment operation in ultrasound treatment in the prior art.

To achieve the above and other related objects, the present disclosure provides a water path arrangement structure for an ultrasound treatment device, including an ultrasound treatment head, a water supply pipeline, and a water receiving tray, wherein the ultrasound treatment head is provided with a cavity configured to accommodate a breast for treatment and having a cavity inner wall with an arc surface, a first connecting channel in communication with the water supply pipeline is disposed at a bottom of the ultrasound treatment head, and during ultrasound treatment, a water medium enters the cavity from the bottom of the ultrasound treatment head and overflows from a top of the cavity of the ultrasound treatment head, and the water receiving tray is disposed around a periphery of the ultrasound treatment head and configured to receive the water medium overflowing from the cavity of the ultrasound treatment head.

Beneficial effects of the present disclosure lie in that: the ultrasound treatment head is directly used as a carrier for containing the water, so that water consumption and pipeline arrangement are reduced, tangling and interference are avoided, a simple structure and a compact layout are obtained, and the treatment operation becomes simpler, more convenient, and more flexible.

Optionally, the first connecting channel is in communication with the water supply pipeline through a pipe socket, the pipe socket is provided with an internal cavity flow channel, a water inlet end of the pipe socket is connected to the water supply pipeline, a water outlet end of the pipe socket passes through the water receiving tray to extend into the first connecting channel and is in sealed connection with the ultrasound treatment head in a relatively rotatable manner, and the internal cavity flow channel is in communication with the cavity.

Optionally, the pipe socket is further provided with a water return cavity below the water receiving tray and configured to receive the water medium discharged from the water receiving tray, and a water baffle extending into the water return cavity is provided at a bottom of the water receiving tray in a downward protruding manner and configured to block a splash of the water medium discharged from the water receiving tray.

Optionally, the pipe socket has a cylindrical structure, the internal cavity flow channel is disposed in a center of the pipe socket, and an upper portion of the pipe socket extends into the first connecting channel to fit with the bottom of the ultrasound treatment head in a sealed and rotatable manner; and a water receiving tank is disposed on a lower outer wall of the pipe socket to form the water return cavity, the water receiving tank includes a bottom plate and a tubular sidewall, the sidewall surrounds a periphery of the pipe socket and is spaced apart from the pipe socket, and the bottom plate is connected between the sidewall and the outer wall of the pipe socket.

Optionally, a three-way pipe joint is installed at the water inlet end of pipe socket, with three interfaces connected to the water supply pipeline, the water inlet end of the pipe socket, and a drain pipeline, respectively.

Optionally, the water receiving tray includes an annular bottom plate, with an outer ring baffle around an outer ring of the annular bottom plate and an inner ring baffle around an inner ring of the annular bottom plate, the annular bottom plate or the inner ring baffle is connected to the bottom of the ultrasound treatment head, and a drain hole for draining the water medium from the water receiving tray is provided in the annular bottom plate between the outer ring baffle and the inner ring baffle.

Optionally, a second connecting channel in sealed communication with the first connecting channel is provided at a center of the water receiving tray, the first connecting channel is connected to the water supply pipeline through the second connecting channel, and a drain port is provided in the second connecting channel.

Optionally, a liquid level sensor and a buffer pipeline configured to protect the liquid level sensor are installed at the water supply pipeline.

Optionally, the ultrasound treatment head is a spherical sound collection treatment head, and the cavity inner wall of the ultrasound treatment head is an indent spherical surface.

The beneficial effects of adopting the above optional schemes lie in that: while openings in the ultrasound treatment head are reduced, feeding and draining of the water to/from the ultrasound treatment head can be implemented more conveniently without any pipeline, thereby avoiding tangling of pipelines.

The present disclosure further provides an ultrasound treatment device, including the water path arrangement structure for an ultrasound treatment device as described above, and a monitoring probe with an acquisition end on a sidewall or bottom in the cavity of the ultrasound treatment head; wherein when the acquisition end of the monitoring probe is on the bottom in the cavity of the ultrasound treatment head, an installation end of the monitoring probe extends out along the first connecting channel to pass through the water receiving tray and to be connected to the water receiving tray in a sealed and relatively rotatable manner.

Beneficial effects of the present disclosure lie in that: uniform and stable flow and replacement of the water medium are achieved and the water consumption is reduced, flexible rotation is realized and the monitoring probe can be adaptively installed on the sidewall or bottom of the ultrasound treatment head according to the treatment requirements, which facilitates the monitoring and treatment, and improves the treatment effect.

### BRIEF DESCRIPTION OF DRAWINGS

FIG. 1 is a schematic structural diagram of an embodiment of a water path arrangement structure for an ultrasound treatment device according to the present disclosure; and
FIG. 2 is a schematic structural diagram of another embodiment of a water path arrangement structure for an ultrasound treatment device according to the present disclosure.

### List of reference numerals:

- 1: monitoring probe;
- 2: breast;
- 3: ultrasound treatment head;
- 301: cavity inner wall;
- 302: first connecting channel;
- 4: water receiving tray;
- 401: water baffle;
- 402: drain hole;
- 403: second connecting channel;
- 404: drain port;
- 5: pipe socket;
- 501: water return cavity;
- 502: internal cavity flow channel;
- 503: drain pipe joint;
- 6: probe mount;
- 7: three-way pipe joint
- 701: water outlet interface;
- 702: water inlet interface;
- 8: water supply pipeline;
- 9: liquid level sensor;
- 901: detection water inlet; and
- 902: buffer pipeline.

### DETAIL DESCRIPTION OF EMBODIMENTS

The following describes implementations of the present disclosure by way of specific examples, and other advantages and effects of the present disclosure will be readily apparent to those skilled in the art from the disclosure herein. The present disclosure can also be implemented or applied in other and different specific implementations, and various details in the description can be modified or changed without departing from the spirit and scope of the present disclosure based on different views or applications.

It should be noted that the terms "upper", "lower", "left", "right", "middle", "one" and the like used in the description are for clarity of description only instead of limiting the implementation scope of the present disclosure, and any change or adjustment of the relative relationship between the terms without any substantial technical change should also be regarded as falling into the implementation scope of the present disclosure.

Before describing embodiments of the present disclosure in detail, an application environment of the present disclosure will be described first. The technology of the present disclosure is mainly applied to ultrasound treatment, in particular to a water path arrangement structure for an ultrasound treatment device. The present disclosure aims to solve the problems of large water consumption, inconvenient rotation operation of the ultrasound treatment head, complex pipelines, interference of pipelines during rotation of the ultrasound treatment head, poor treatment effect and the like in the ultrasound treatment process.

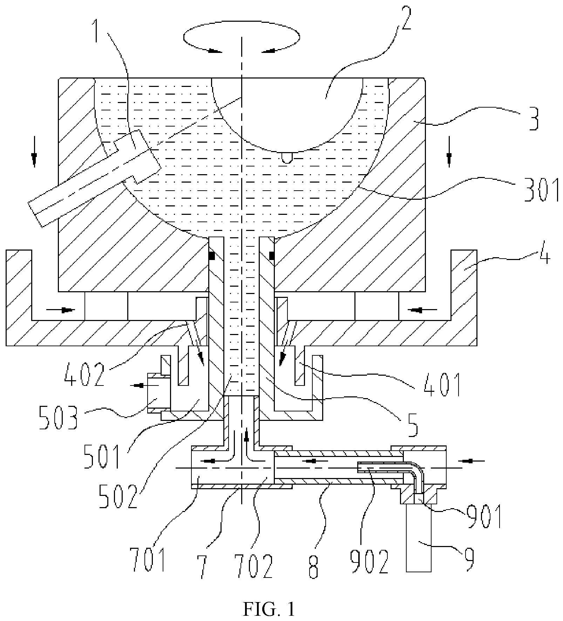

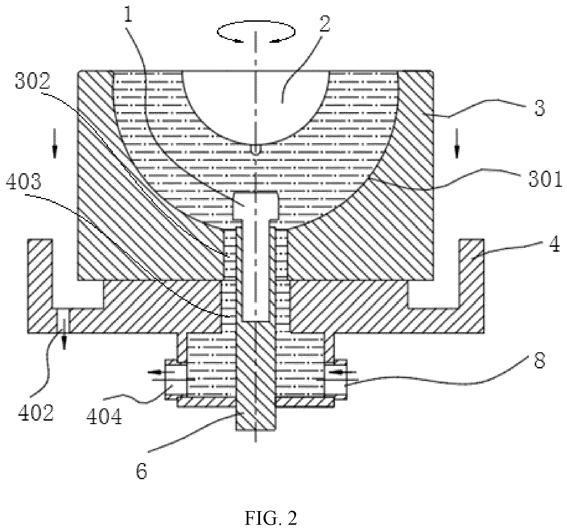

As shown in Figs. 1 and 2, the water path arrangement structure for an ultrasound treatment device of the present disclosure includes an ultrasound treatment head 3, a water supply pipeline 8, and a water receiving tray 4. The ultrasound treatment head 3 is provided with a cavity configured to accommodate a breast 2 for treatment and having a cavity inner wall 301 with an arc surface, and a first connecting channel 302 in communication with the water supply pipeline 8 is disposed at a bottom of the ultrasound treatment head 3. During ultrasound treatment, a water medium enters the cavity of the ultrasound treatment head from the bottom of the ultrasound treatment head 3 and overflows from a top of the cavity of the ultrasound treatment head 3. The water receiving tray 4 is disposed around a periphery of the ultrasound treatment head 3 and configured to receive the water medium overflowing from the cavity of the ultrasound treatment head. The ultrasound treatment head 3 is directly used as a carrier for containing the water medium, so that the water consumption is reduced. The cooperation of the ultrasound treatment head 3 and the water receiving tray 4 not only reduces the pipeline arrangement, but also makes the feeding and draining of water continuous and stable. In this case, the ultrasound treatment head 3 will not be disturbed by pipeline tangling even when the ultrasound treatment head needs to be flexibly rotated, so that the ultrasound treatment head can be stably used in ultrasound treatment, and the treatment effect can be guaranteed.

As shown in FIG. 1, in an exemplary embodiment, the first connecting channel is in communication with the water supply pipeline 8 through a pipe socket 5 which is provided with an internal cavity flow channel 502. A water inlet end of the pipe socket is connected to the water supply pipeline, a water outlet end of the pipe socket passes through the water receiving tray 4 to extend into the first connecting channel and is in sealed connection with the ultrasound treatment head 3 in a relatively rotatable manner. The internal cavity flow channel 502 is in communication with the cavity, so that the ultrasound treatment head 3 can be flexibly rotated around the pipe socket 5 to drive the monitoring probe 1 on the ultrasound treatment head 3 to rotate together with the ultrasound treatment head 3, so as to implement monitoring of different areas.

As shown in FIG. 1, in an exemplary embodiment, the pipe socket 5 is further provided with a water return cavity 501 below the water receiving tray 4 and configured to receive a water medium discharged from the water receiving tray 4. A water baffle 401 extending into the water return cavity 501 is provided at a bottom of the water receiving tray 4 in a downward protruding manner and configured to block a splash of the water medium discharged from the water receiving tray 4.

As shown in FIG. 1, in an exemplary embodiment, the pipe socket 5 has a cylindrical structure, and the internal cavity flow channel 502 is disposed in a center of the pipe socket 5. By means of the internal cavity flow channel 502, not only an external water medium can be introduced into the ultrasound treatment head 3, but also a water medium inside the ultrasound treatment head 3 can be thoroughly drained from the ultrasound treatment head 3 as required. During ultrasound treatment, the water medium enters from the bottom of the ultrasound treatment head 3 and overflows from the top, so that the water medium has a stable flow direction, and the cooling capability is improved. An upper portion of the pipe socket 5 extends into the first connecting channel to fit with the bottom of the ultrasound treatment head 3 in a sealed and rotatable manner. In this embodiment, a groove is provided around an outer sidewall of the pipe socket of the cylindrical structure, and a sealing ring is installed in the groove and tightly attached to the groove and the first connecting channel in a sealed manner, so that the pipe socket 5 can be rotatably connected to the ultrasound treatment head 3 while the connection tightness is ensured, and leakage of the water medium from the ultrasound treatment head along the rotatable joint can be avoided. A water receiving tank is disposed on a lower outer wall of the pipe socket 5. The water receiving tank forms the water return cavity 501, and is provided with a water outlet. The water receiving tank includes a bottom plate and a tubular sidewall, the sidewall surrounds a periphery of the pipe socket 5 and is spaced apart from the pipe socket 5, and the bottom plate is connected between the sidewall and the outer wall of the pipe socket 5. In this embodiment, the bottom plate may be an annular plate, an inner ring edge of which is connected to a lower outer wall of the pipe socket 5, and an outer ring edge of which is connected to a bottom of the tubular sidewall. The pipe socket 5 and the water receiving tank may form an integrated structure, resulting in a simple structure, simple production and manufacture, as well as low cost. A drain pipe joint 503 is installed at the water outlet of the water receiving tank, and the drain pipe joint 503 may be installed on the tubular sidewall to discharge the water medium from the water return cavity 501. At least one drain pipe joint 503 is provided, and the specific number may be set as required. The drain pipe joint 503 is connected to an external drain pipe to discharge the water medium to a designated position.

As shown in FIG. 1, in an exemplary embodiment, a three-way pipe joint 7 is installed at the water inlet end of pipe socket 5, with three interfaces thereof connected to the water supply pipeline 8, the water inlet end of the pipe socket, and a drain pipeline, respectively, that is, a water outlet interface 701 connected to the drain pipeline, a water inlet interface 702 connected to the water supply pipeline 8, and an interface connected to the water inlet end of the pipe socket 5.

As shown in Figs. 1 and 2, in some exemplary embodiments, the water receiving tray 4 includes an annular bottom plate, with an outer ring baffle around an outer ring of the annular bottom plate and an inner ring baffle around an inner ring of the annular bottom plate. The annular bottom plate or the inner ring baffle is connected to the bottom of the ultrasound treatment head, and a drain hole 402 for draining the water medium from the water receiving tray 4 is provided in the annular bottom plate between the outer ring baffle and the inner ring baffle.

As shown in FIG. 1, in an exemplary embodiment, the annular bottom plate is connected to the bottom of the ultrasound treatment head, the water medium in the water receiving tray 4 is introduced into the water return cavity 501 through the drain hole 402, and a water baffle 401 is disposed at a bottom of the annular bottom plate. The water outlet end of the pipe socket 5 passes through a central hole of the annular bottom plate, and an outer sidewall of the water outlet end of the pipe socket 5 may be in clearance fit with the central hole to reduce friction and enable relatively flexible rotation. In this embodiment, both the outer ring baffle and the inner ring baffle are provided to protrude upward, and the drain hole is inclined to penetrate the annular bottom plate. With such a structural design, a buffering effect is achieved when the water medium is discharged, and when the water medium impacts on the outer sidewall of the pipe socket, a splash of the water medium can be prevented with the outer sidewall of the pipe socket and the water baffles in cooperation with each other. The annular bottom plate may be fixedly connected to the ultrasound treatment head 3 so that the water receiving tray 4 can be rotated with the ultrasound treatment head 3, and the annular bottom plate has an outer contour larger than the ultrasound treatment head 3 so that the water receiving tray 4 can sufficiently carry a medium overflowing from the ultrasound treatment head 3. With such a structural layout of the water receiving tray and the pipe socket, the water medium can stably flow into the water return cavity 501 without any connection through a pipeline, thereby reducing the difficulty of structural matching and sealing as well as the difficulty of production and manufacture.

As shown in FIG. 2, in an exemplary embodiment, the inner ring baffle is connected to the bottom of the ultrasound treatment head, and a second connecting channel 403 in sealed communication with the first connecting channel 302 is provided at a center of the water receiving tray 4. The first connecting channel 302 is connected to the water supply pipeline 8 through the second connecting channel 403, and a drain port 404 is formed in the second connecting channel 403. In this embodiment, the drain port 404 is disposed on the water receiving tray 4, connected to the second connecting channel 403, and configured to thoroughly drain the water medium from the cavity of the ultrasound treatment head 3. The water receiving tray 4 and the ultrasound treatment head 3 may be two parts in fixed connection, so that as long as a joint of the first connecting channel 302 and the second connecting channel 403 is sealed, the water medium can be prevented from leaking along the joint, and the production, processing and manufacture are relatively simple. Alternatively, the water receiving tray 4 and the ultrasound treatment head 3 may form an integrated structure, and the first connecting channel 302 and the second connecting channel 403 form a continuous channel to obtain better sealing performance.

As shown in Figs. 1 and 2, in some exemplary embodiments, the water supply pipeline 8 is provided with a liquid level sensor 9 and a buffer pipeline 902 configured to protect the liquid level sensor 9, and a water level in the ultrasound treatment head 3 is monitored with the liquid level sensor 9. The buffer pipeline 902 may have an L type structure, with one end installed at a detection water inlet 901 of the liquid level sensor 9 for buffering, which can protect the liquid level sensor 9 from being impacted by an inlet water pressure and reduce openings in the ultrasound treatment head, and with the other end toward a three-way pipe joint 7 so that an external medium can stably flow into the three-way pipe joint 7 along the buffer pipeline.

As shown in Figs. 1 and 2, in some exemplary embodiments, the ultrasound treatment head 3 may be a spherical sound collection treatment head, and the cavity inner wall 301 of the ultrasound treatment head is an indent spherical surface.

As shown in Figs. 1 and 2, the present disclosure provides an ultrasound treatment device, including a monitoring probe 1, and the water path arrangement structure for an ultrasound treatment device as described in any of the above exemplary embodiments. An acquisition end of the monitoring probe 1 is located on a sidewall or bottom in a cavity of the ultrasound treatment head 3 to meet different treatment requirements.

As shown in FIG. 1, in an exemplary embodiment, the acquisition end of the monitoring probe 1 is located on the sidewall in the cavity of the ultrasound treatment head 3, and during ultrasound treatment, an axis of the ultrasound treatment head 3 may be arranged to be eccentric or coincident with an axis of the breast 2 according to the treatment requirement. In this embodiment, the axis of the ultrasound treatment head 3 is arranged to be eccentric with the axis of the breast 2, and the ultrasound treatment head drives the monitoring probe 1 to rotate around the axis of the ultrasound treatment head together to monitor different quadrants of the breast 2, so that the monitoring range is wider and clearer, observation is facilitated, and the treatment effect is favorably provided.

As shown in FIG. 2, in an exemplary embodiment, the acquisition end of the monitoring probe 1 is located on the bottom in the cavity of the ultrasound treatment head 3, and an installation end of the monitoring probe extends out along the first connecting channel 302 to pass through the second connecting channel 403 on the water receiving tray 4 and to be connected to the water receiving tray 4 in a sealed and relatively rotatable manner. The monitoring probe 1 may be connected to the water receiving tray in a sealed and relatively rotatable manner through a probe mount 6. The installation end of the monitoring probe 1 is installed on the probe mount 6, an upper end of the probe mount 6 is located in the first connecting channel 302 and the second connecting channel 403, and a lower end of the probe mount 6 passes through the water receiving tray 4. The probe mount 6 may be connected to the water receiving tray in a sealed and rotatable manner through soft sealing, so that the probe mount 6 can be moved up and down, rotated, swung, or perform other movements under driving of an external power mechanism to adjust a position of the monitoring probe 1 to implement monitoring of different positions. As a result, the ultrasound treatment head 3 does not need not to be rotated, and the structure is simplified. In this embodiment, since the axes of the ultrasound treatment head 3, the breast 2, and the monitoring probe 1 are coincided, the monitoring probe 1 can be moved independently to adjust the monitored areas.

As shown in Figs. 1 and 2, in some exemplary embodiments, the monitoring probe 1 may be a camera or a B-mode ultrasound probe.

According to the water path arrangement structure for an ultrasound treatment device and the ultrasound treatment device of the present disclosure, the cavity of the ultrasound treatment head is used as a carrier for containing the water medium while playing a role in treatment and medium containing. Further, by a mutually cooperation of layouts of the ultrasound treatment head and the water receiving tray, resource consumption is saved, feeding and draining of the water to/from the ultrasound treatment head can be implemented without any pipeline, which avoids pipeline tangling and interference, and the monitoring probe can flexibly adjust the monitoring range according to different monitoring requirements, so that a simple structure and a compact layout are obtained, the difficulty of production, manufacture and installation, as well as the cost, are reduced, and the safety performance and treatment effect are improved.

The foregoing embodiments are merely for illustration of the principles and utilities of the present disclosure, but are not intended to limit the present disclosure. Those skilled in the art can modify or change the above embodiments without departing from the spirit and scope of the present disclosure. Accordingly, it is intended that all equivalent modifications or changes which may be made by those of ordinary skill in the art without departing from the spirit and scope of the present disclosure are covered by the appended claims.

## Claims

1. A water path arrangement structure for an ultrasound treatment device, **characterized in**: comprising an ultrasound treatment head, a water supply pipeline, and a water receiving tray, wherein the ultrasound treatment head is provided with a cavity configured to accommodate a breast for treatment and having a cavity inner wall with an arc surface, a first connecting channel in communication with the water supply pipeline is disposed at a bottom of the ultrasound treatment head, and during ultrasound treatment, a water medium enters the cavity from the bottom of the ultrasound treatment head and overflows from a top of the cavity of the ultrasound treatment head, and the water receiving tray is disposed around a periphery of the ultrasound treatment head and configured to receive the water medium overflowing from the cavity of the ultrasound treatment head.

2. The water path arrangement structure for an ultrasound treatment device according to claim 1, **characterized in that**: the first connecting channel is in communication with the water supply pipeline through a pipe socket, the pipe socket is provided with an internal cavity flow channel, a water inlet end of the pipe socket is connected to the water supply pipeline, a water outlet end of the pipe socket passes through the water receiving tray to extend into the first connecting channel and is in sealed connection with the ultrasound treatment head in a relatively rotatable manner, and the internal cavity flow channel is in communication with the cavity.

3. The water path arrangement structure for an ultrasound treatment device according to claim 2, **characterized in that**: the pipe socket is further provided with a water return cavity below the water receiving tray and configured to receive a water medium discharged from the water receiving tray, and a water baffle extending into the water return cavity is provided at a bottom of the water receiving tray in a downward protruding manner and configured to block a splash of the water medium discharged from the water receiving tray.

4. The water path arrangement structure for an ultrasound treatment device according to claim 3, **characterized in that**: the pipe socket has a cylindrical structure, the internal cavity flow channel is disposed in a center of the pipe socket, and an upper portion of the pipe socket extends into the first connecting channel to fit with the bottom of the ultrasound treatment head in a sealed and rotatable manner; and a water receiving tank is disposed on a lower outer wall of the pipe socket to form the water return cavity, the water receiving tank comprises a bottom plate and a tubular sidewall, the sidewall surrounds a periphery of the pipe socket and is spaced apart from the pipe socket, and the bottom plate is connected between the sidewall and the outer wall of the pipe socket.

5. The water path arrangement structure for an ultrasound treatment device according to claim 2, **characterized in that**: a three-way pipe joint is installed at the water inlet end of pipe socket, with three interfaces thereof connected to the water supply pipeline, the water inlet end of the pipe socket, and a drain pipeline, respectively.

6. The water path arrangement structure for an ultrasound treatment device according to claim 1, **characterized in that**: the water receiving tray comprises an annular bottom plate, with an outer ring baffle around an outer ring of the annular bottom plate and an inner ring baffle around an inner ring of the annular bottom plate, the annular bottom plate or the inner ring baffle is connected to the bottom of the ultrasound treatment head, and a drain hole for draining the water medium from the water receiving tray is provided in the annular bottom plate between the outer ring baffle and the inner ring baffle.

7. The water path arrangement structure for an ultrasound treatment device according to claim 1, **characterized in that**: a second connecting channel in sealed communication with the first connecting channel is provided at a center of the water receiving tray, the first connecting channel is connected to the water supply pipeline through the second connecting channel, and a drain port is provided in the second connecting channel.

8. The water path arrangement structure for an ultrasound treatment device according to claim 1, **characterized in that**: a liquid level sensor and a buffer pipeline configured to protect the liquid level sensor are installed at the water supply pipeline.

9. The water path arrangement structure for an ultrasound treatment device according to claim 1, **characterized in that**: the ultrasound treatment head is a spherical sound collection treatment head, and the cavity inner wall of the ultrasound treatment head is an indent spherical surface.

10. An ultrasound treatment device, comprising the water path arrangement structure for an ultrasound treatment device according to any one of claims 1 to 9, and **characterized in**: further comprising a monitoring probe with an acquisition end on a sidewall or bottom in the cavity of the ultrasound treatment head; wherein when the acquisition end of the monitoring probe is on the bottom in the cavity of the ultrasound treatment head, an installation end of the monitoring probe extends out along the first connecting channel to pass through the water receiving tray and to be connected to the water receiving tray in a sealed and relatively rotatable manner.
